# EUROPEAN PATENT SPECIFICATION

(11) **EP 4 210 644 B1**
(45) Date of publication and mention of the grant of the patent: **30.04.2025**
(21) Application number: 21794621.9
(22) Date of filing: 01.09.2021
(51) Int. Cl.: A61F 9/00

(54) **SURGICAL FIXATION TOOL**
CHIRURGISCHES FIXIERWERKZEUG
OUTIL DE FIXATION CHIRURGICAL

(30) Priority: 08.09.2020 US 202063075365 P
(43) Date of publication of application: 19.07.2023
(73) Proprietor: Everads Therapy Ltd, 6971039 Tel Aviv (IL)
(72) Inventor: DRORI, Hagay, 6219501 Tel Aviv (IL); SINAI, Nir, 1792000 Alon Hagalil (IL); SMULIAN, Doron, 2622052 Haifa (IL); DAILY, David, 4636431 Herzliya (IL); RADAY, Lior, 7915200 Bror Hayil (IL)
(74) Representative: Wright, Howard Hugh Burnby
(86) International application number: PCT/IB2021/057988
(87) International publication number: WO 2022/053912

(56) References cited:
- WO-A1-2019/202603
- CN-U- 208 876 973
- CN-U- 209 770 657
- US-A1- 2013 253 416

## Description

### FIELD OF THE INVENTION

The present invention is related generally to surgical devices and particularly to a surgical fixation tool for use in procedures, such as in ophthalmology.

### BACKGROUND OF THE INVENTION

PCT Application WO 2019/202603 describes a device, inter alia, for injection of a medication into an interlayer, such as the suprachoroidal layer of the eye. The device includes a needle having a size transitional portion terminating in a sharp needle distal tip. The device also includes an elongated tissue separator that is shiftable in a needle lumen. An actuator shifts the tissue separator in the needle lumen between a first position, in which the separator distal tip is fixedly positioned proximally to the needle distal tip, and a second position in which the separator distal tip is fixedly positioned a predetermined distance distally from the needle distal tip.

That device also includes an organ docking device which includes an anchoring member and an anchoring actuator. The anchoring member is configured to anchor to the target sclera portion. The anchoring member includes two anchors peripherally spaced and symmetrically opposing each other. Each anchor has a sharp anchor tip for penetrating into the target sclera portion. The anchors project perpendicularly from a base surface formed by two anchoring member legs, which are spaced with each other and symmetrically arranged so that the needle distal tip can pass between the anchors.

US-2013/253416A1 describes subretinal delivery of therapeutic compositions.

CN-208876973U describes an eye watch fixing device.

WO-2019/202603A1 describes a device for injecting a substance into an interlayer of a body tissue or organ.

CN-209770657U describes a sclera fixator and kit.

### SUMMARY

The present invention is defined by claim 1 and seeks to provide an improved surgical fixation tool for use in surgical procedures, such as ophthalmic procedures, such as but not limited to, injection of a substance into an interlayer, such as the suprachoroidal layer of the eye.

For use in ophthalmology, the fixation tool may be used to manipulate, position and/or stabilize the eye. For example, the fixation tool may be used as a counterforce against forces applied during a surgical procedure, injection, diagnostic procedure, etc. The fixation tool may also be used to rotate or translate the ocular tissue during a surgical procedure.

The fixation tool may be used to stretch and hold the conjunctiva to allow for easy penetration into/under the sclera. Likewise, the fixation tool may be used to stretch and hold the sclera for easy penetration into/under the sclera by surgical tools (needle, knife or other tools). The fixation tool may be used to shift the orientation of the eyeball to ensure comfortable and optimal access for ophthalmic procedures. The fixation tool allows all of these procedures to be done atraumatically without perforating the sclera or other sensitive layers or tissues.

There is provided a surgical fixation tool including at least one fixation member which protrudes from a leg of a proximal grasping portion, the at least one fixation member including a needle with a sharp tip at a distal end of a needle shaft, wherein the needle protrudes distally from a support structure assembled on the needle shaft and which includes one or more sloped support surfaces that are sloped with respect to the needle shaft.

### BRIEF DESCRIPTION OF THE DRAWINGS

The present invention will be better understood with reference to the following drawings and description. The components in the figures are not necessarily to scale, emphasis instead being placed upon illustrating the principles of the invention.
Fig. 1 is a simplified pictorial illustration of a fixation tool, constructed and operative in accordance with an embodiment of the invention, with a protector member covering fixation members;
Fig. 2 is a simplified pictorial illustration of the fixation tool with the protector member removed, exposing the fixation members;
Fig. 3 is a simplified planar-view illustration of the fixation tool;
Fig. 4 is a simplified planar-view illustration of a distal end of the fixation tool, with the protector member removed, exposing the fixation members;
Fig. 4A is an enlarged view of the distal end of the fixation tool with the fixation members;
Fig. 5 is a simplified pictorial illustration of the fixation tool being used together with a needle and separator tool in an ophthalmic procedure, such as creating a suprachoroidal passage for introducing a substance therein;
Fig. 6A is an illustration of a tip angle and tip length of the fixation member;
Fig. 6B is an illustration of a support angle of support structure of the fixation member;
Fig. 6C is an illustration of a width W of the fixation member, Fig. 6C being rotated 90° relative to Fig. 6B;
Figs. 7A, 7B and 7C are illustrations of different fixation members and their support structure, in accordance with different embodiments of the invention; and
Fig. 8A and 8B are simplified illustrations of the fixation tool, placed a distance (e.g., 3 mm) from the limbus and placed at the limbus.

### DETAILED DESCRIPTION

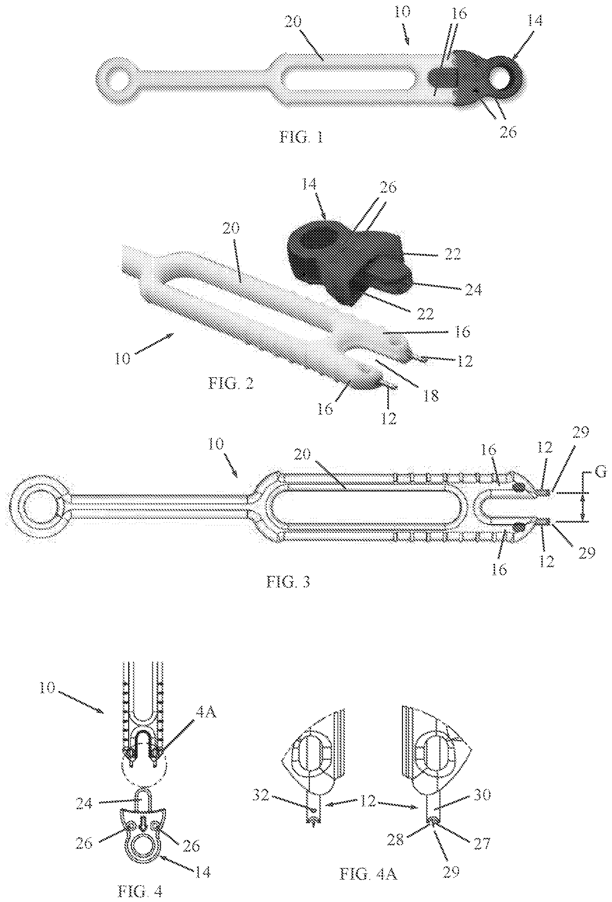

Reference is now made to Figs. 1-3, which illustrate a surgical fixation tool 10, constructed and operative in accordance with the invention. The surgical fixation tool 10 includes at least one fixation member 12, whose structure is described hereinbelow. Fixation tool 10 may include a protector member 14 that can cover the one or more fixation members 12 before use, to protect the sharp end of the fixation member 12 from being damaged or contaminated and protect the user from accidentally being injured by the fixation member 12.

Although the invention can be carried out with just one fixation member 12, in the illustrated and preferred embodiment there are more than one fixation members 12, and in particular and in accordance with the invention a pair of fixation members 12, each of which protrudes from a leg 16. Accordingly in the illustrated embodiment, and according to the invention, there are a pair of legs 16 separated by a (e.g., U-shaped) channel 18. The legs 16 extend from a proximal grasping portion 20 of the tool 10.

The protector member 14 may be formed with at least one hollow receiving member 22, one for each of the fixation members 12. A tongue 24 may protrude axially outwards between the hollow receiving members 22 and may be arranged to move into channel 18 of the tool 10. The protector member 14 may be formed with at least one window 26 (also seen in Fig. 4) for viewing the at least one fixation member 12 while the protector member 14 covers the fixation members 12. In this manner, even before removing protector member 14, the user can visually inspect the integrity of the fixation members 12 before use.

Reference is now made to Fig. 4A. Each fixation member 12 includes a needle 28 that includes a needle shaft 27 with a sharp tip 29 at a distal end of the needle shaft 27. Needle 28 may or may not be hollow and can be generally round/tubular or have any other crosssection, size and shape. Needle 28 protrudes distally from support structure 30, which has multiple sloped support surfaces as explained below. The support surfaces 30 are sloped with respect to the needle shaft 27. Referring again to Fig. 3, the tips 29 of the fixation members 12 may be separated by a distance G, which may be, without limitation, 7 mm-10 mm, or 2-24 mm. The separation or gap G between the fixation members 12 may enable the fixation members 12 to resist rotational movement of the eye and apply tension to the conjunctiva and sclera. Additionally, the separation G between the fixation members 12 may serve as a slot to indicate the correct access point for a procedure. Additionally, the separation G between the fixation members 12 may provide clearance to manipulate the fixation tool in vicinity of surgical tools or structures working within the separation or gap G.

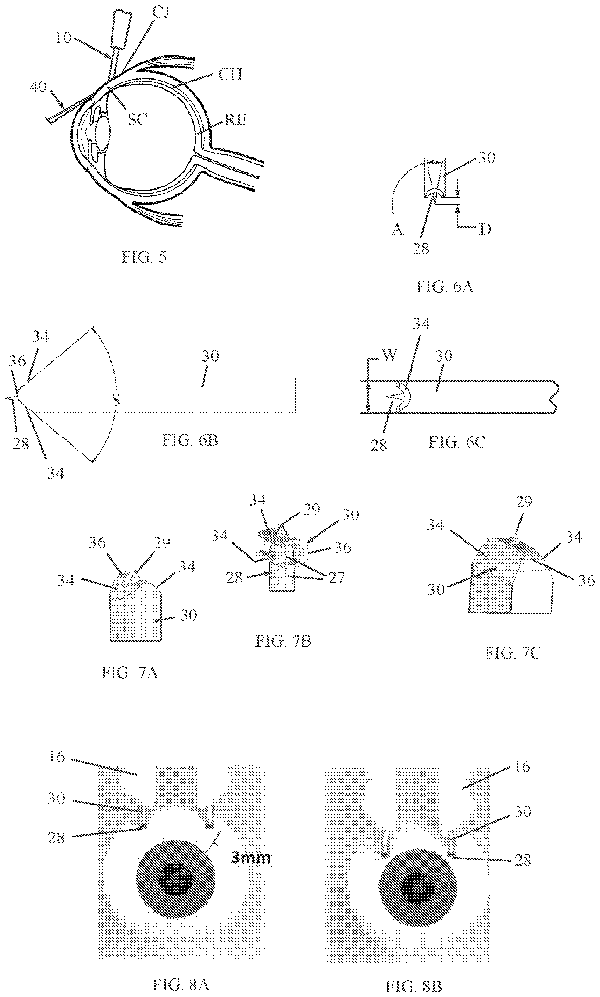

Reference is now made to Fig. 6A. The needle 28 may be a pencil point that subtends an angle A, which is preferably but not necessarily 18-22°, or 7°-150°. This shape helps penetrate and hold tissue without cutting or tearing. The needle 28 may alternatively be a chisel point. The length D of needle 28 that protrudes outwards from the support structure 30 is intended to anchor the tissue without perforating the target tissue, and is preferably but not necessarily 0.4 mm, or 0.2-1.2 mm. The full diameter of needle 28 is preferably but not necessarily 0.3 mm or 0.05- 2 mm.

The gap G between tips 29 of needles 28 is fixed, but could, in non-claimed embodiments useful for understanding the invention, be flexible or adjustable, such as by mounting legs 16 on a gear rack or groove and the like, so the legs 16 can be moved towards and away from each other.

The needles 28 may include a sensor 32 (Fig. 4A) that senses contact with the tissue or a parameter of the tissue. Sensor 32 may be, without limitation, an optical, force, impedance, temperature, resistance sensor and others. Sensor 32 may be adjusted to measure intraocular pressure.

The needle 28 may be fixed with respect to support structure 30, or alternatively, may be spring loaded, so that the needles 28 may be actuated or adjusted relative to support structure 30.

Support structure 30 may be fixed with respect to legs 16, or alternatively, may be spring loaded, so that the support structure 30 may be actuated or adjusted relative to legs 16.

Support structure 30 may be actuated by an actuator to shield or expose needle 28.

Reference is now made to Fig. 6B and to Figs. 7A-7C, which illustrate examples of the support structure 30. The support structure 30 includes two or more sloping shoulders 34 that are joined at a curved portion 36. The angle S (Fig. 6B) between the sloping shoulders may be, but is not necessarily, 80°, and is 10°-180°. The sloping shoulders 34 enable manipulating the angle of the fixation tool relative to the ocular tissue after the needle tips have engaged the ocular tissue, without having to remove the needle tips and re-position them and without losing the grip force to the tissue. This minimizes injuring the ocular tissue (only one placement of the needle tips is necessary). This also provides greater and easier access to, and view of, the ocular surface between the tips of the tool. In this manner, the tool can apply counter forces to multiple forces of varying directions during the ophthalmic procedure.

The support structure 30 also provides control over tip penetration depth into the ocular tissue.

There are many ways to produce the sharp tip 29 and the support structure 30. For example, the tips can be produced as an independent component or integral to the support structure 30. The support structure 30 can be produced from a tube (Fig. 7A), in which the tube longitudinal central axis is concentric and parallel to the longitudinal central axis of the needle. Angled surfaces may be ground or otherwise formed on the tube wall. In Fig. 7B, the longitudinal central axis of the tube is perpendicular to the needle. Holes are formed in the tube to accommodate the needle tip. The round shape of the tube provides the sloping shoulders 34. The protective shoulder can be produced from any other shape and suitable material.

Reference is now made to Figs. 8A and 8B. The total length of the projections (support structure 30 and needles 28 that protrude from the legs 16) may be used as an indicator to measure distances; e.g., 3 mm distance from the limbus (Fig. 8B) and afterwards placing the needles 3mm from the limbus (Fig. 8A).

Reference is now made to Fig. 5. The fixation tool 10 may be used in surgical procedures, such as ophthalmic procedures, such as but not limited to, injection of a substance into an interlayer, such as the suprachoroidal layer of the eye, such as in the procedure described in PCT Application WO 2019/202603, or any other ophthalmic procedure that requires scleral or conjunctival fixation. Fig. 5 shows fixation tool fixing the conjunctiva CJ so that a needle and separator tool 40 (described in PCT Application WO 2019/202603) can create a suprachoroidal tunnel between the choroid CH and the sclera SC. (RE is the retina.) The fixation tool 10 can apply counter forces to multiple forces of varying directions during the ophthalmic procedure. For example, the separator tool 40 can apply forces tangential to the ocular tissue, or forces at any acute or obtuse angle.

## Claims

1. A surgical fixation tool (10) for scleral or conjunctival fixation comprising:
a proximal grasping portion (20) comprising a pair of legs (16) and **characterised by** a single fixation member (12) protruding from each of said legs (16), each of said fixation members (12) comprising a needle (28) with a sharp tip (29) at a distal end of a needle shaft (27), wherein said needle (28) protrudes distally from a support structure (30) assembled on said needle shaft (27) that comprises one or more sloped support surfaces (34, 36) that are sloped with respect to said needle shaft (27),
wherein said one or more sloped support surfaces comprise more than one sloped surfaces that are joined at a curved portion (36) and wherein an angle (S) between said sloped support surfaces is 10°-180°; and
wherein said legs (16) are separated by a channel (18) and said tips (29) of said fixation members (12) are separated by a gap (G) and wherein said gap (G) is fixed.

2. The surgical fixation tool (10) according to claim 1, further comprising a protector member (14) for covering said fixation members (12).

3. The surgical fixation tool (10) according to claim 2, wherein said protector member (14) is formed with at least one window (26) for viewing said at least one fixation member (12) while said protector member (14) covers said fixation members (12).

4. The surgical fixation tool (10) according to claim 1, further comprising a protector member (14) for covering said fixation members (12), wherein said protector member (14) is formed with hollow receiving members (22), one for each of said fixation members (12), and a tongue (24) that protrudes axially outwards between said hollow receiving members (22).

5. The surgical fixation tool (10) according to claim 1, wherein said fixation members (12) comprise a sensor (32) configured to sense contact with a tissue or a parameter of a tissue.

6. The surgical fixation tool (10) according to claim 1, wherein said gap (G) is 2-24 mm.

7. The surgical fixation tool (10) according to claim 1, wherein said needle (28) comprises a pencil point that subtends an angle of 7°-150°.

8. The surgical fixation tool (10) according to claim 1, wherein a length (D) of said needle (28) that protrudes distally from said support structure (30) is 0.2-1.2 mm.

9. The surgical fixation tool (10) according to claim 1, wherein said needle (28) is fixed with respect to said support structure (30).

10. The surgical fixation tool (10) according to claim 1, wherein said needle (28) is adjustable with respect to said support structure (30).

11. The surgical fixation tool (10) according to claim 1, wherein said support structure (30) is fixed with respect to said legs (16).

12. The surgical fixation tool (10) according to claim 1, wherein said support structure (30) is adjustable with respect to said legs (16).

## Patentansprüche

1. Chirurgisches Fixierwerkzeug (10) zur skleralen oder konjunktivalen Fixierung, umfassend:
einen proximalen Greifabschnitt (20), der ein Paar von Schenkeln (16) umfasst und durch ein einzelnes Fixierungselement (12) gekennzeichnet ist, das von jedem der Schenkel (16) vorsteht, wobei jedes der Fixierungselemente (12) eine Nadel (28) mit einer scharfen Spitze (29) an einem distalen Ende eines Nadelschafts (27) umfasst, wobei die Nadel (28) distal aus einer auf dem Nadelschaft (27) montierten Stützstruktur (30) hervorsteht, die eine oder mehrere geneigte Stützflächen (34, 36) umfasst, die in Bezug auf den Nadelschaft (27) geneigt sind,
wobei die eine oder die mehreren geneigten Stützflächen mehr als eine geneigte Fläche umfassen, die an einem gekrümmten Abschnitt (36) verbunden sind, und wobei ein Winkel (S) zwischen den geneigten Auflageflächen 10° bis 180° beträgt; und
wobei die Schenkel (16) durch einen Kanal (18) und die Spitzen (29) der Fixierungselemente (12) durch einen Spalt (G) voneinander getrennt sind, und wobei der Spalt (G) fest ist.

2. Chirurgisches Fixierwerkzeug (10) nach Anspruch 1, weiter umfassend ein Schutzelement (14) zum Abdecken der Fixierungselemente (12).

3. Chirurgisches Fixierwerkzeug (10) nach Anspruch 2, wobei das Schutzelement (14) mit mindestens einem Fenster (26) zum Betrachten des mindestens einen Fixierungselements (12) ausgebildet ist, während das Schutzelement (14) die Fixierungselemente (12) abdeckt.

4. Chirurgisches Fixierwerkzeug (10) nach Anspruch 1, weiter umfassend ein Schutzelement (14) zum Abdecken der Fixierungselemente (12), wobei das Schutzelement (14) mit hohlen Aufnahmeelementen (22), einem für jedes der Fixierungselemente (12), und einer Zunge (24) ausgebildet ist, die zwischen den hohlen Aufnahmeelementen (22) axial nach außen vorsteht.

5. Chirurgisches Fixierwerkzeug (10) nach Anspruch 1, wobei die Fixierungselemente (12) einen Sensor (32) umfassen, der so konfiguriert ist, dass er den Kontakt mit einem Gewebe oder einen Parameter eines Gewebes erfasst.

6. Chirurgisches Fixierwerkzeug (10) nach Anspruch 1, wobei der Spalt (G) 2 bis 24 mm beträgt.

7. Chirurgisches Fixierwerkzeug (10) nach Anspruch 1, wobei die Nadel (28) eine Stiftspitze aufweist, die einen Winkel von 7°-150° einschließt.

8. Chirurgisches Fixierwerkzeug (10) nach Anspruch 1, wobei eine Länge (D) der Nadel (28), die distal aus der Stützstruktur (30) herausragt, 0,2 bis 1,2 mm beträgt.

9. Chirurgisches Fixierwerkzeug (10) nach Anspruch 1, wobei die Nadel (28) in Bezug auf die Stützstruktur (30) fest ist.

10. Chirurgisches Fixierwerkzeug (10) nach Anspruch 1, wobei die Nadel (28) in Bezug auf die Stützstruktur (30) einstellbar ist.

11. Chirurgisches Fixierwerkzeug (10) nach Anspruch 1, wobei die Stützstruktur (30) in Bezug auf die Beine (16) fest ist.

12. Chirurgisches Fixierwerkzeug (10) nach Anspruch 1, wobei die Stützstruktur (30) in Bezug auf die Beine (16) einstellbar ist.

## Revendications

1. Outil de fixation chirurgical (10) pour fixation sclérale ou conjonctivale comprenant :
une partie de préhension proximale (20) comprenant une paire de branches (16) et **caractérisé par** un seul élément de fixation (12) qui fait saillie à partir de chacune desdites branches (16),
chacun desdits éléments de fixation (12) comprenant une aiguille (28) avec une pointe acérée (29) au niveau d'une extrémité distale d'une tige d'aiguille (27),
dans lequel ladite aiguille (28) fait saillie de manière distale à partir d'une structure de support (30) assemblée sur ladite tige d'aiguille (27) qui comprend une ou plusieurs surfaces de support inclinées (34, 36) qui sont inclinées par rapport à ladite tige d'aiguille (27),
dans lequel lesdites une ou plusieurs surfaces de support inclinées comprennent plusieurs surfaces inclinées qui sont jointes au niveau d'une partie incurvée (36) et dans lequel un angle (S) entre lesdites surfaces de support inclinées est compris entre 10° et 180° ; et
dans lequel lesdites branches (16) sont séparées par un canal (18) et lesdites pointes (29) desdits éléments de fixation (12) sont séparées par un espace (G) et dans lequel ledit espace (G) est fixe.

2. Outil de fixation chirurgical (10) selon la revendication 1, comprenant en outre un élément protecteur (14) pour recouvrir lesdits éléments de fixation (12).

3. Outil de fixation chirurgical (10) selon la revendication 2, dans lequel ledit élément protecteur (14) est formé avec au moins une fenêtre (26) pour visualiser ledit au moins un élément de fixation (12) pendant que ledit élément protecteur (14) recouvre lesdits éléments de fixation (12).

4. Outil de fixation chirurgical (10) selon la revendication 1, comprenant en outre un élément protecteur (14) pour recouvrir lesdits éléments de fixation (12), dans lequel ledit élément protecteur (14) est formé d'éléments de réception creux (22), un pour chacun desdits éléments de fixation (12), et d'une languette (24) qui fait saillie axialement vers l'extérieur entre lesdits éléments de réception creux (22).

5. Outil de fixation chirurgical (10) selon la revendication 1, dans lequel lesdits éléments de fixation (12) comprennent un capteur (32) configuré pour détecter un contact avec un tissu ou un paramètre d'un tissu.

6. Outil de fixation chirurgical (10) selon la revendication 1, dans lequel ledit espace (G) est compris entre 2 et 24 mm.

7. Outil de fixation chirurgical (10) selon la revendication 1, dans lequel ladite aiguille (28) comprend une pointe de crayon qui sous-tend un angle de 7° à 150°.

8. Outil de fixation chirurgical (10) selon la revendication 1, dans lequel une longueur (D) de ladite aiguille (28) qui fait saillie de manière distale à partir de ladite structure de support (30) est comprise entre 0,2 et 1,2 mm.

9. Outil de fixation chirurgical (10) selon la revendication 1, dans lequel ladite aiguille (28) est fixe par rapport à ladite structure de support (30).

10. Outil de fixation chirurgical (10) selon la revendication 1, dans lequel ladite aiguille (28) est réglable par rapport à ladite structure de support (30).

11. Outil de fixation chirurgical (10) selon la revendication 1, dans lequel ladite structure de support (30) est fixe par rapport auxdites branches (16).

12. Outil de fixation chirurgical (10) selon la revendication 1, dans lequel ladite structure de support (30) est réglable par rapport auxdites branches (16).
